Europäisches Patentamt

European Patent Office

Office européen des brevets

(19)

(11) Numéro de publication: **0 506 565 B1**

(12) **FASCICULE DE BREVET EUROPEEN**

(45) Date de publication de fascicule du brevet: **07.12.94**  (51) Int. Cl.5: **A23K 1/165**, A61K 31/565

(21) Numéro de dépôt: **92400833.7**

(22) Date de dépôt: **26.03.92**

(54) **Nouvelle utilisation de stéroides 4,9,11-triéniques dans des compositions destinées à l'administration chez la dinde.**

(30) Priorité: **28.03.91 FR 9103777**

(43) Date de publication de la demande:
**30.09.92 Bulletin 92/40**

(45) Mention de la délivrance du brevet:
**07.12.94 Bulletin 94/49**

(84) Etats contractants désignés:
**AT BE CH DE DK FR GB IT LI LU NL SE**

(56) Documents cités:
**EP-A- 0 272 976**
**FR-A- 2 168 207**

**NUTRITION REPORTS INTERNATIONAL vol. 31, no. 1, Janvier 1985, LOS ALTOS, CALIF., USA pages 59 - 65; D.V.MAURICE ET AL.: 'Response of turkeys to the anabolic agent trenbolone acetate'**

(73) Titulaire: **ROUSSEL-UCLAF**
**35, Boulevard des Invalides**
**F-75007 Paris (FR)**

(72) Inventeur: **Grandadam, Jean André**
**56, Avenue Gabriel Péri**
**F-94100 Saint Maur des Fosses (FR)**

(74) Mandataire: **Tonnellier, Marie-José et al**
**111, route de Noisy**
**B.P. no 9**
**F-93230 Romainville (FR)**

Rank Xerox (UK) Business Services
(3.10/3.09/3.3.3)

EP 0 506 565 B1

**Description**

La présente invention concerne une nouvelle utilisation de stéroïdes 4,9,11-triéniques.

L'invention a pour objet l'utilisation chez la dinde de l'acétate de trenbolone, caractérisée en ce que l'acétate de trenbolone est incorporé à la nourriture à raison de 0,5 à 10 ppm en poids par poids de nourriture.

L'acétate de trenbolone est un produit connu pour ses propriétés anabolisantes (cf Merck Index, 10ème édition, rubrique 9402).

Le brevet FRA 2168207 décrit l'administration de stéroïdes 4,9,11-triéniques par voie orale chez la volaille.

Le brevet EP A 0 272976 envisage l'administration d'acétate de trenbolone en combinaison avec un béta adrénergique par voie orale en administration animale notamment chez la volaille.

L'acétate de trenbolone a déjà été administré en implant chez la dinde (cf Maurice D.V. Jones et al. Nutr. Rep. Int. 31(1) 1985, 59-66).

Il est connu que l'acétate de trenbolone administré par voie orale chez divers animaux notamment les bovins a une activité relativement faible (cf A.G. Rico et V. Burgat-Sagaze dans Anabolisants en Production Animale, Symposium tenu à l'O.I.E. Paris 15,17 février 1983).

De ce fait, l'acétate de trenbolone est utilisé sous forme d'implant dans l'élevage des bovins par exemple.

On vient de découvrir qu'il était possible d'obtenir d'excellents résultats chez la dinde en administrant par voie orale l'acétate de trenbolone comme le montrent les résultats de tests biologiques joints en annexe.

L'administration par voie orale évite les manipulations stressantes et permet la modulation des doses selon l'âge des animaux au moment du traitement.

De plus, aucun effet secondaire n'a été observé :
- les résidus plasmatiques de TBA à 10, 12, 14, 16 et 18 semaines par exemple sont inférieurs à la limite de dosage.
- l'évolution du taux des transaminases plasmatiques (GOT et GPT) n'est pas modifiée par rapport aux témoins.

De plus, les animaux ne présentent aucune anomalie dans leur comportement locomoteur.

L'invention a plus spécialement pour objet l'utilisation caractérisée en ce que l'acétate de trenbolone est incorporé à la nourriture pour les dindes à raison de l à 4 ppm en poids par poids de nourriture.

On a obtenu de très bons résultats en utilisant par exemple de 0,2 à 3 mg d'acétate de trenbolone par jour et par dinde à partir de la dixième semaine de traitement et notamment un très bon indice de consommation (IC), c'est à dire, le rapport du poids total de nourriture consommée par l'animal sur le gain total de poids, comme le montrent les résultats de tests biologiques joints en annexe.

Les exemples suivants illustrent l'invention sans toutefois la limiter.

**EXEMPLES DE COMPOSITIONS POUR L'UTILISATION SELON L'INVENTION :**

- On a préparé des compositions renfermant : aliment du commerce vendu sous la marque SANDERS (2ème âge) auquel on ajoute 2,5 ppm d'acétate de trenbolone.
- On a préparé des compositions renfermant 1,5 ppm d'acétate de trenbolone.
- On a préparé des compositions renfermant 4 ppm d'acétate de trenbolone.

**ESSAI BIOLOGIOUE.**

L'essai a été effectué sur des dindes femelles âgées de 10 semaines.
Les animaux sont répartis en 2 groupes :
- un groupe témoin de 37 animaux,
- un groupe de 37 animaux recevant de l'acétate de trenbolone dans leur nourriture.

Les dindes reçoivent une quantité rationnée de nourriture renfermant l'acétate de trenbolone puis sont nourries ad libitum.

2

| Action de l'acétate de trenbolone " per os " chez la dinde femelle | PM | GPM | %var. | I.C. | Stat. |
|---|---|---|---|---|---|
| **Période préexpérimentale** | | | | | |
| *9ème semaine* | | | | | |
| Témoins | 3262 | | | | |
| Traités | 3361,5 | | | | |
| *10ème semaine* | | | | | |
| Témoins | 3964,4 | | | 2,13 | |
| Traités | 4097,9 | | | 2 | |
| **Période expérimentale 1 : Acétate de trenbolone 0,7 mg/Jour/dinde** | | | | | |
| *11ème semaine* | | | | | |
| Témoins | 4700,1 | 1438,1 | | 2,2 | |
| Traités | 4967,6 | 1606,1 | *12%* | 2,05 | ** |
| *12ème semaine* | | | | | |
| témoins | 5249,2 | 1947,2 | | 2,45 | |
| Traités | 5665,2 | 2303,7 | *18%* | 2,05 | *** |
| *13ème semaine* | | | | | |
| Témoins | 5856,5 | 2594,5 | | 2,71 | |
| Traités | 6268,7 | 2907,2 | *12%* | 2,54 | *** |
| *14ème semaine* | | | | | |
| Témoins | 6749,8 | 3487,8 | | 2,84 | |
| Traités | 7200,9 | 3839,4 | *10%* | 2,66 | *** |
| *15ème semaine* | | | | | |
| Témoins | 7426 | 4164 | | 3,04 | |
| Traités | 7825,1 | 4463,6 | *7%* | 2,66 | ** |
| *16ème semaine* | | | | | |
| | 8135,9 | 4873,9 | | 3,26 | |
| | 8619,6 | 5258 | *8%* | 3,1 | ** |
| **Période expérimentale 2 : Acétate de trenbolone 1,4 mg /jour/dinde** | | | | | |
| *17ème semaine* | | | | | |
| Témoins | 8771,3 | 5509,3 | | 3,54 | |
| Traités | 9369,7 | 6008,1 | *9%* | 3,33 | *** |
| *18ème semaine* | | | | | |
| Témoins | 9239,2 | 5977,2 | | 3,74 | |
| Traités | 10012,8 | 6651,2 | *11%* | 3,45 | *** |
| *19éme semaine* | | | | | |
| Témoins | 9728,6 | 6466,6 | | 3,87 | |
| Traités | 10559,4 | 7197,8 | *11%* | 3,59 | *** |

*PM = poids moyen en grammes*
*GPM= Gain de poids cumulé*
*% variation= % de l'augmentation de GPM des traités par rapport aux témoins*
*I.C. = indice de consommation*
*stat. = test t : **différence de poids significative à 1 p 100 , ***à 1 p1000*

On calcule le poids moyen des dindes par pesée individuelle chaque semaine.
On calcule le gain de poids moyen
et l'indice de consommation c'est-à-dire

$$\frac{\text{le poids total de nourriture que l'animal a reçu}}{\text{gain de poids total}}$$

Les résultats sont résumés dans le tableau ci-dessus, ainsi que dans les courbes jointes en annexe.
- Courbe 1 : évolution pondérale des dindes femelles de 9 à 19 semaines.
- Courbe 2 : évolution de la différence entre les indices de consommation cumulés témoins-traités.
- Courbe 3 : évolution de la différence entre les gains de poids moyens des animaux traités par rapport aux animaux témoins.

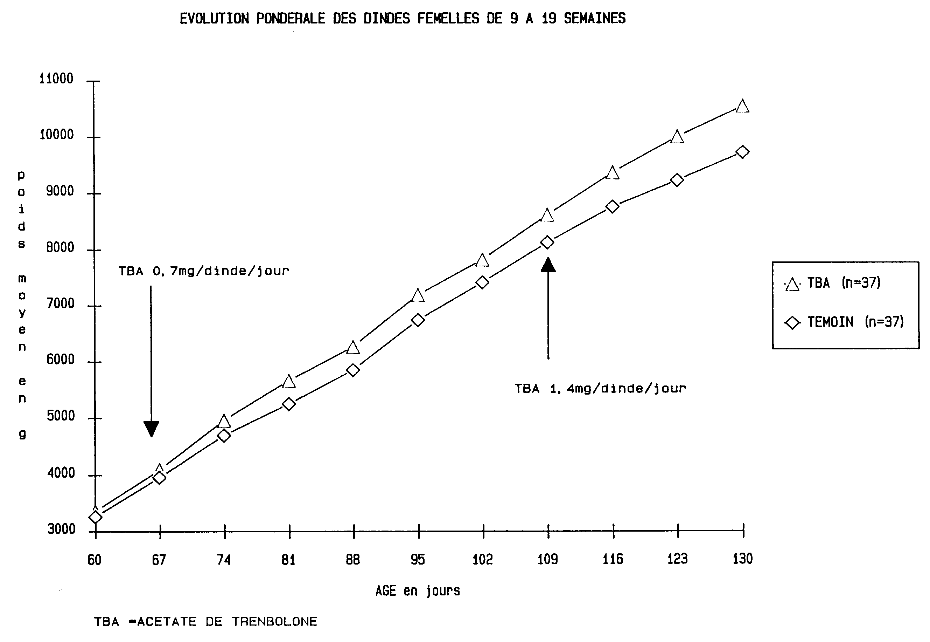

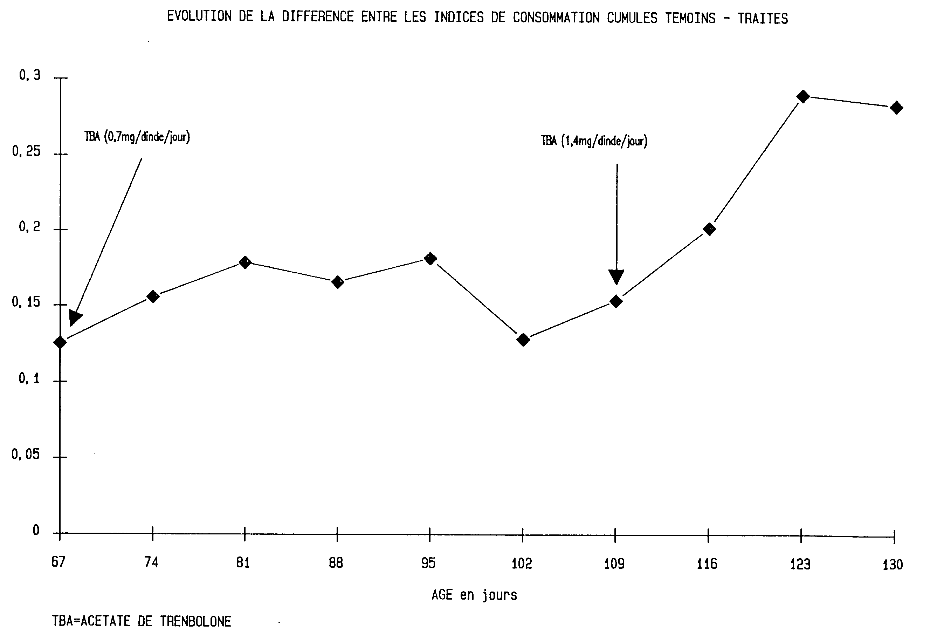

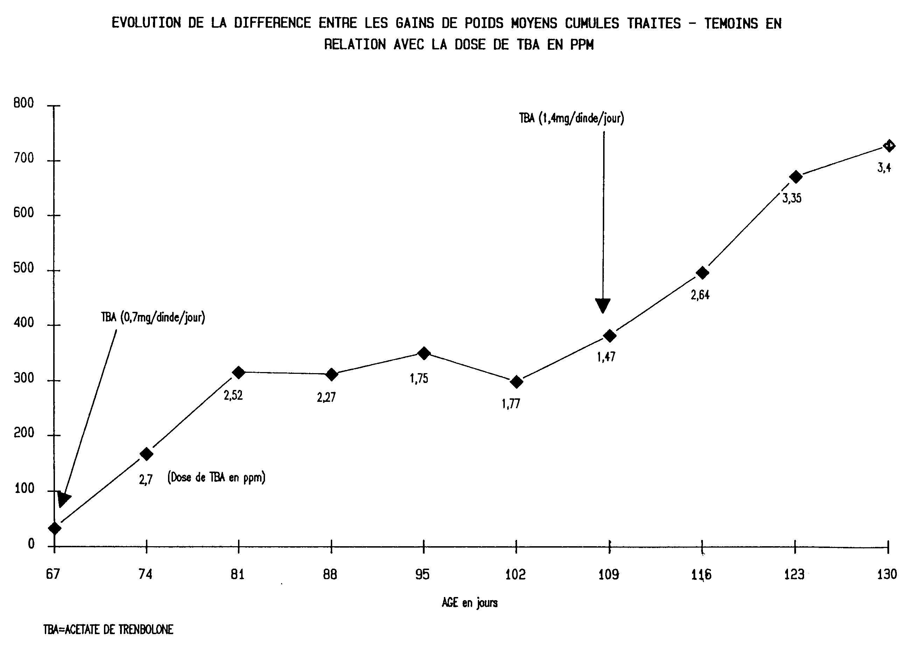

**Revendications**

1. Utilisation chez la dinde de l'acétate de trenbolone, caractérisée en ce que l'acétate de trenbolone est incorporé à la nourriture à raison de 0,5 à 10 ppm en poids par poids de nourriture.

2. Utilisation selon la revendication 1, caractérisée en ce que l'acétate de trenbolone est incorporé à la nourriture à raison de 1 à 4 ppm en poids par poids de nourriture.

**Claims**

1. Use in turkeys of trenbolone acetate, characterized in that trenbolone acetate is incorporated in the food at a rate of 0.5 to 10 ppm by weight per weight of food.

2. Use according to claim 1, characterized in that trenbolone acetate is incoroporated in the food at a rate of 1 to 4 ppm by weight per weight of food.

**Patentansprüche**

1. Verwendung von Trenbolonacetat bei Truthühnern, dadurch gekennzeichnet, daß das Trenbolonacetat in das Futter in einem Verhältnis von 0,5 bis 10 ppm in Gewicht pro Gewicht Futter eingebracht wird.

2. Verwendung nach Anspruch 1, dadurch gekennzeichnet, daß das Trenbolonacetat in das Futter in einem Verhältnis von 1 bis 4 ppm in Gewicht pro Gewicht Futter eingebracht wird.

EVOLUTION PONDERALE DES DINDES FEMELLES DE 9 A 19 SEMAINES

EVOLUTION DE LA DIFFERENCE ENTRE LES INDICES DE CONSOMMATION CUMULES TEMOINS - TRAITES

TBA (1,4mg/dinde/jour)

TBA (0,7mg/dinde/jour)

AGE en jours

TBA=ACETATE DE TRENBOLONE

0,3  0,25  0,2  0,15  0,1  0,05  0

67  74  81  88  95  102  109  116  123  130

EVOLUTION DE LA DIFFERENCE ENTRE LES GAINS DE POIDS MOYENS CUMULES TRAITES – TEMOINS EN
RELATION AVEC LA DOSE DE TBA EN PPM

TBA (1,4mg/dinde/jour)

TBA (0,7mg/dinde/jour)

3,4

3,35

2,64

1,47

1,75

1,77

2,52

2,27

2,7   (Dose de TBA en ppm)

AGE en jours

TBA=ACETATE DE TRENBOLONE

EP 0 506 565 B1